## Europäisches Patentamt

## European Patent Office

## Office européen des brevets

(11) Veröffentlichungsnummer: **0 152 963 B1**

## EUROPÄISCHE PATENTSCHRIFT

(45) Veröffentlichungstag der Patentschrift: **03.07.91**

(21) Anmeldenummer: **85101993.5**

(22) Anmeldetag: **22.02.85**

(51) Int. Cl.⁵: **A61N 1/40**, A61N 1/36, A61N 2/04

(54) **Elektrotherapiegerät.**

(30) Priorität: **23.02.84 DE 3406565**

(43) Veröffentlichungstag der Anmeldung:
**28.08.85 Patentblatt 85/35**

(45) Bekanntmachung des Hinweises auf die
Patenterteilung:
**03.07.91 Patentblatt 91/27**

(84) Benannte Vertragsstaaten:
**AT BE CH DE FR GB IT LI NL SE**

(56) Entgegenhaltungen:
| | |
|---|---|
| AT-A- 312 793 | DE-A- 1 171 095 |
| DE-A- 2 952 850 | DE-A- 3 340 974 |
| FR-A- 862 014 | GB-A- 1 026 701 |
| GB-A- 1 602 354 | US-A- 2 936 762 |
| US-A- 3 543 762 | |

(73) Patentinhaber: **Kraus, Werner, Dipl.-Ing.
Augustenstrasse 41
W-8000 München 2(DE)**

(72) Erfinder: **Kraus, Werner, Dipl.-Ing.
Augustenstrasse 41
W-8000 München 2(DE)**

(74) Vertreter: **von Bezold, Dieter, Dr. et al
Dr. Dieter von Bezold Dipl.-Ing. Peter Schütz
Dipl.-Ing. Wolfgang Heusler Brienner Strasse
52
W-8000 München 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die vorliegende Erfindung betrifft ein Elektrotherapiegerät, bei dem sowohl niederfrequente Magnetfelder mit Frequenzen unter 30 Hz als auch hochfrequente elektromagnetische Felder mit Frequenzen über 100 kHz, vorzugsweise 1 MHz und mehr zur Anwendung gelangen.

Die Ergebnisse experimenteller und klinischer Studien bestätigen die Hypothese, daß die Heilwirkungen und die Förderung des Gewebewachstums sowie der Gewebergeneration durch nicht-thermische, sehr niederfrequente sinusförmige Magnetfelder auf Ladungstrennungsprozessen im Proto- und Zytoplasma sowie auf Elektronentransferprozessen in der Enzymkette der Sauerstoffatmung beruhen. Voraussetzung für die Stimulation der Sauerstoffatmung der Zelle ist, daß Sauerstoff in ausreichendem Maße zur Verfügung steht. Defizite des Sauerstoffangebots in der Zelle treten auf, wenn die Blutzirkulation im Gewebe, sei es durch Verletzung der Gefäße oder durch pathologische Veränderungen gestört ist oder, wie bei den malignen Entartungen eines Tumors, die Sauerstoff-Permeabilität und -Affinität der Zell-Innenmembran vermindert ist.

Einrichtungen zum Behandeln von erkrankten oder atrophierten Geweben mit niederfrequenten magnetischen Wechselfeldern oder mit einer Kombination von niederfrequenten elektrischen und magnetischen Wechselfeldern sind z.B. aus DE-A-2116869 und DE-A-2314573 bekannt.

Die Mängel der Sauerstoffversorgung durch überlange Transportwege (entsprechendes gilt auch für die sogenannten bradytrophen Gewebe, die kein eigenes Kapillarsystem haben) sowie durch verminderte Sauerstoff-Membran-Permeabilität können auch physikalisch durch Erhöhung des interkapillaren Sauerstoffpartialdruckes, vor allem aber technisch einfacher durch eine thermisch bewirkte Verstärkung der Sauerstoff-Diffusion reduziert oder vermieden werden. Zum diesbezüglichen Stand der Technik gehören die zahlreichen Diathermie- und Hyperthermieverfahren, die die thermische Diffusion und auch die nicht katalysierten biochemischen Reaktionen durch eine Temperaturerhöhung im Gewebe beschleunigen können, die mit Hilfe von Mikrowellen, Kurzwellen oder Infrarotstrahlung bewirkt werden kann. Die relativ geringe Temperaturbelastbarkeit lebenden Gewebes beschränkt diese Verfahren jedoch auf Temperaturen unter etwa 43,5° C. Die Wirkung der Hochfrequenz-Wärmebehandlung läßt sich auch durch das z. B. aus DE-A-2952850, US-A-3543762 und GB-A-1026701 bekannte Verfahren, die Hochfrequenz mit einer niederfrequenten oder impulsförmigen Schwingung in der Amplitude zu modulieren, nicht merklich verbessern.

Das aus GB-A-1602354 bekannte Verfahren, Gewebe mit pulsmodulierten hochfrequenten Faradisierungsströmen zu behandeln, ist für die obigen Indikationen weder bestimmt noch geeignet.

Die vorliegende Erfindung löst die Aufgabe, die Wirksamkeit eines Elektrotherapiegerätes mit einem Generatorteil, der einen Hochfrequenzgenerator zum Erzeugen von thermisch wirksamen ersten Schwingungen mit einer Frequenz über 100 kHz enthält, ferner mit einem Applikatorteil, der einen mit dem Hochfrequenzgenerator gekoppelten Applikator enthält und mit einem Generator für ein niederfrequentes Signal zu verbessern, dadurch, daß der Generator für das niederfrequente Signal eine sinus- oder pulsdruckwellenförmige zweite Schwingung mit einer Frequenz unter 30 Hz liefert und mit einer für die Frequenz der zweiten Schwingungen bemessenen Niederfrequenz-Applikatorspule gekoppelt ist.

Bei dem vorliegenden Elektrotherapiegerät werden also hochfrequente Schwingungen und niederfrequente Schwingungen in Kombination zur Einwirkung gebracht.

Weiterbildungen und vorteilhafte Ausgestaltungen des erfindungsgemäßen Therapiegerätes sind Gegenstand von Unteransprüchen.

Es hat sich überraschenderweise gezeigt, daß durch kombinierte Einwirkung von Wärme einerseits und nicht-thermischen, niederfrequenten elektromagnetischen Feldern andererseits eine Heil- und Stimulationswirkung erreicht werden kann, die erheblich über die Summe der Wirkungen der Einzelmaßnahmen hinausgeht. Durch das nicht-thermische niederfrequente elektromagnetische Feld, dessen Frequenz vorzugsweise unter 25 Hz liegt und das vorzugsweise ein Temperaturäquivalent von weniger als 1/100° C hat, kann außerdem die thermische Überlastung und Schädigung des wärmebehandelten Gewebes wesentlich reduziert werden.

Im folgenden werden Ausführungsbeispiele der vorliegenden Elektrotherapiegeräte unter Bezugnahme auf die Zeichnungen näher erläutert.

Es zeigen:

Fig. 1     eine vereinfachte Darstellung eines ersten Ausführungsbeispieles des vorliegenden Elektrotherapiegerätes;

Fig. 2     eine schematische Darstellung eines zweiten Ausführungsbeispieles des vorliegenden Elektrotherapiegerätes und

Fig. 3     eine graphische Darstellung des zeitlichen Verlaufes von niederfrequenten und hochfrequenten Schwingungen, die mit dem vorliegenden Elektrotherapiegerät zur Einwirkung gebracht werden können.

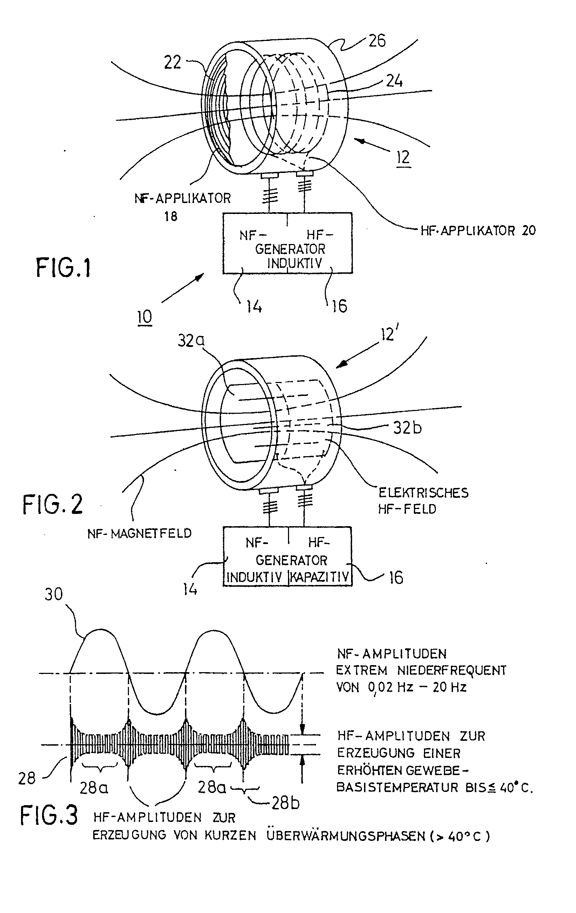

Das Elektrotherapiegerät gemäß Fig. 1 enthält im wesentlichen einen Generatorteil 10 und einen Applikatorteil 12. Der Generatorteil 10 enthält einen Niederfrequenz- oder NF-Generator 14 sowie einen Hochfrequenz oder HF-Generator 16. In entsprechender Weise enthält der Applikatorteil einen NF-Applikator 18 und einen HF-Applikator 20.

Bei dem Ausführungsbeispiel gemäß Fig. 1 enthalten sowohl der NF-Applikator 18 als auch der HF-Applikator 20 jeweils eine Solenoidspule 22 bzw. 24, die im wesentlichen koaxial in einem gemeinsamen, hohlzylinderförmigen Gehäuse 26 aus einem geeigneten, verlustfreien Kunststoff untergebracht sind. Es ist bekanntlich vorteilhaft, wenn die Feldlinien des niederfrequenten Feldes in Längsrichtung der Haupt-Blutgefäße des zu behandelnden Körperteils verlaufen.

Der NF-Generator arbeitet mit einer vorzugsweise einstellbaren Frequenz, die vorteilhafterweise im Frequenzbereich zwischen etwa 0,02 und 20 Hz liegt. Der NF-Generator ist in Kombination mit der NF-Solenoidspule 22 so bemessen, daß die Feldstärke des niederfrequenten magnetischen Wechselfeldes im Inneren des Applikator-Gehäuses 26 vorzugsweise zwischen etwa 1 und 50 mT liegt.

Der HF-Generator 16 kann mit irgendeiner der für medizinische Zwecke vorgesehenen, genormten Frequenzen arbeiten, z. B. mit einer Frequenz von 27,5 MHz. Die Ausgangsleistung des HF-Generators 16 ist in üblicher Weise einstellbar, so daß sie der jeweiligen Situation angepaßt werden kann.

Der HF-Generator 16 enthält vorzugsweise einen Amplitudenmodulator, der die Amplitude der HF-Schwingung 28 (Fig. 3) mit einer Modulationsschwingung zu modulieren gestattet, die vorzugsweise mit der vom NF-Generator 14 gelieferten NF-Schwingung 30 synchronisiert ist. Beispielsweise kann, wie es in Fig. 3 dargestellt ist, die Amplitude der HF-Schwingung näherungsweise invers zum Betrag der Amplitude der NF-Schwingung so moduliert werden, daß sich Bereiche 28a mit einer im wesentlichen konstanten Amplitude, die zur Erzeugung einer erhöhten Gewebe-Basis-Temperatur bis etwa 40 °C bemessen ist, mit relativ kurz dauernden Bereichen 28b abwechseln, die bezüglich der Nulldurchgänge der NF-Schwingung zentriert sind und Amplituden aufweisen, die kurze Überwärmungsphasen mit Gewebetemperaturen über 40 °C erzeugen. Die Einhüllende der HF-Schwingung kann in den Bereichen 28b beispielsweise etwa dreieckig sein, wie es in Fig. 3 dargestellt ist, und das Verhältnis der Dauer der Bereiche 28a zur Dauer der Bereiche 28b kann beispielsweise 3 : 1 oder auch wesentlich mehr betragen, was bis zu einem gewissen Grade von der Frequenz der NF-Schwingung abhängt.

Die NF-Schwingung kann eine reine Sinusschwingung sein oder einen Verlauf haben, der der systolischen-diastolischen Blutdruckwelle einer elastischen Arterie gleicht. Die NF-Schwingung kann ferner mit dem Puls der zu behandelnden Person synchronisierbar sein. Die relative Phasenlage der NF-Schwingung und der Modulation der HF-Schwingung ist vorteilhafterweise veränderbar.

Das Verhältnis der Amplitude der HF-Schwingung in den Bereichen 28a zur Maximalamplitude in den Bereichen 28b kann 3 : 1 oder wesentlich mehr betragen und so gewählt werden, daß der durch die Amplituden in den Bereichen 28a erzeugten erhöhten Gewebe-Basistemperatur kurzzeitig Wärmepulse oder -Schocks erzeugt werden, die dann jeweils während der nachfolgenden Bereiche 28a durch die Blutzirkulation wieder aufgelöst werden.

Die Ausführungsform gemäß Fig. 2 unterscheidet sich von der gemäß Fig. 1 darin, daß der Applikatorteil 12' zwei einander gegenüberliegende kapazitive Elektroden 32a, 32b zur Applikation des HF-Feldes enthält. Im übrigen kann das Elektrotherapiegerät gemäß Fig. 2 ebenso ausgebildet sein, wie das oben beschriebene Elektrotherapiegerät gemäß Fig. 1.

Anstelle des induktiven oder kapazitiven HF-Applikators gemäß Fig. 1 und 2 können bei Verwendung höherer Frequenzen, z.B. 3000 MHz, auch Hornstrahler oder andere bekannte Mikrowellen-Applikatoren verwendet werden.

Anstelle der hohlzylinderförmigen, starren Applikatorteile 12 oder 12' können auch verformbare Applikatoren oder bügelförmige (U-förmige) Spulenkonfigurationen verwendet werden, wie sie für NF-Applikatoren aus DE-A-21 16 869 und DE-A-23 14 573 und DE-A-23 14 573 bekannt sind.

Die Generatoren sind mit den jeweiligen Applikatoren durch geeignete, flexible Leitungen, z.B. Koaxialkabel, verbunden. Die Nennleistung des HF-Generators kann beispielsweise 1 kW für Dauerbetrieb und 10 kW für Kurzzeit- oder Impulsbetrieb betragen.

## Ansprüche

1.  Elektrotherapiegerät mit einem Generatorteil, der einen Hochfrequenzgenerator (16) zum Erzeugen von thermisch wirksamen ersten Schwingungen mit einer Frequenz über 100 kHz enthält, ferner mit einem Applikatorteil, der einen mit dem Hochfrequenzgenerator gekoppelten ersten Applikator (24; 32a, 32b) enthält, und mit einem Generator (14) für ein niederfrequentes Signal, dadurch gekennzeichnet, daß der Generator (14) für das niederfrequente Signal eine sinus- oder pulsdruckwellenförmige zweite Schwingung mit einer Frequenz unter 30 Hz liefert und mit einer für die Frequenz der zweiten Schwingungen bemessenen Niederfrequenz-Applikatorspule (18) gekoppelt ist.

2. Elektrotherapiegerät nach Anspruch 1, dadurch gekennzeichnet, daß der erste Applikator eine für die Frequenz der ersten Schwingungen bemessene Solenoidspule (24) enthält, die mit der Niederfrequenz-Applikatorspule (22) im wesentlichen koaxial in einem hohlzylinderförmigen Gehäuse (26) angeordnet ist.

3. Elektrotherapiegerät nach Anspruch 1, dadurch gekennzeichnet, daß die Niederfrequenz-Applikatorspule (22) in einem hohlzylinderförmigen Gehäuse angeordnet ist und daß der erste Applikator zwei einander gegenüberliegende kapazitive Elektroden (32a, 32b) enthält, die am Gehäuse (26) auf gegenüberliegenden Seiten des Gehäuseinneren angeordnet sind (Fig. 2).

4. Elektrotherapiegerät nach einem der Ansprüche 1 bis 3, dadurch gekennzeichnet, daß der Hochfrequenzgenerator (16) eine Vorrichtung zur Amplitudenmodulation einer von ihm erzeugten Hochfrequenzschwingung (28) mit einer Modulationsschwingung sowie eine Anordnung zur Einstellung einer vorgegebenen Phasenlage zwischen der Modulationsschwingung und der niederfrequenten zweiten Schwingung (30) enthält.

5. Elektrotherapiegerät nach Anspruch 4, dadurch gekennzeichnet, daß die Modulationsschwingung erste Bereiche (28a) relativ niedriger Amplitude zum Erzeugen einer erhöhten Gewebe-Basistemperatur und mit diesen ersten Bereichen (28a) abwechselnde zweite Bereiche (28b) mit relativ großer Amplitude zur Erzeugung von kurzen Überwärmungsphasen enthält.

6. Elektrotherapiegerät nach Anspruch 5, dadurch gekennzeichnet, daß die zweiten Bereiche (28b) im wesentlichen mit den Nulldurchgängen der niederfrequenten zweiten Schwingung (30) zusammenfallen.

7. Elektrotherapiegerät nach einem der Ansprüche 1 bis 6, gekennzeichnet durch eine Vorrichtung zum Synchronisieren des Generators (14) für das niederfrequente Signal mit der Pulswelle eines behandelten Patienten.

8. Elektrotherapiegerät nach einem der Ansprüche 1 bis 7, gekennzeichnet durch eine Vorrichtung zur Modulation des Hochfrequenzgenerators (16) mit der Pulswelle eines behandelten Patienten.

**Claims**

1. Apparatus for electrotherapy with a generator part, which includes a high frequency generator (16) for generating thermally active first oscillations with a frequency above 100 kHz, further with an applicator part, which includes a first applicator (24; 32a, 32b) coupled to the high frequency generator, and with a generator (14) for a low frequency signal, characterized in that the generator (14) for the low frequency signal supplies a second oscillation with a sinusoidal or pulse pressure shaped waveform with a frequency below 30 Hz and is coupled to a low-frequency applicator coil (18) dimensioned for the frequency of the second oscillations.

2. Apparatus for electrotherapy according to claim 1, characterized in that the first applicator includes a solenoid coil (24) dimensioned for the frequency of the first oscillations and which is arranged in a hollow cylindrical housing (26) substantially coaxial with the low frequency applicator coil (22).

3. Apparatus for electrotherapy according to claim 1, characterized in that the low frequency applicator coil (22) is arranged in a hollow cylindrical housing and in that the first applicator comprises two capacitor electrodes (32a, 32b) lying opposite to one another, which are arranged on the housing (26) on opposite sides of the interior of the housing (Figure 2).

4. Apparatus for electrotherapy according to one of claims 1 to 3, characterized in that the high frequency generator (16) includes a device for amplitude modulation of a high frequency oscillation (28) generated thereby with a modulating oscillation, as well as an arrangement for setting a predetermined phase angle between the modulating oscillation and the low frequency second oscillation (30).

5. Apparatus for electrotherapy according to claim 4, characterized in that the modulating oscillation comprises first regions (28a) of relatively low amplitude for generating a raised tissue base temperature and second regions (28b) alternating with these first regions (28a) with relatively high amplitude for the generation of brief over-heating phases.

6. Apparatus for electrotherapy according to claim 5, characterized in that the second regions (28b) coincide substantially with the zero crossings of the low frequency second oscillation (30).

7. Apparatus for electrotherapy according to one of claims 1 to 6, characterized by a device for synchronising the generator (14) for the low frequency signal with the pulse wave of a person under treatment.

8. Apparatus for electrotherapy according to one of claims 1 to 7, characterized by a device for modulation of the high frequency generator (16) with the pulse wave of a person under treatment.

**Revendications**

1. Appareil d'électrothérapie comportant une partie oscillateur qui contient un oscillateur haute fréquence (16) destiné à la production de premières oscillations thermiques efficaces de fréquence supérieure à 100 kHz, ainsi qu'une partie applicateur qui contient un premier applicateur connecté à l'oscillateur haute fréquence (24; 32a, 32b) et un oscillateur (14) destiné à un signal basse fréquence, caractérisé en ce que l'oscillateur (14) destiné au signal basse fréquence fournit une seconde oscillation sinusoïdale ou en forme d'onde de pression du pouls de fréquence inférieure à 30 Hz et est connecté à une bobine d'applicateur basse fréquence dimensionnée en fonction de la fréquence des secondes oscillations.

2. Appareil d'électrothérapie selon la revendication 1, caractérisé en ce que le premier applicateur contient une bobine (24) de solénoïde dimensionnée en fonction de la fréquence des premières oscillations, disposée avec la bobine d'applicateur basse fréquence (22) de façon essentiellement coaxiale dans un boîtier (26) en forme de cylindre creux.

3. Appareil d'électrothérapie selon la revendication 1, caractérisé en ce que la bobine d'applicateur basse fréquence (22) est disposée dans un boîtier en forme de cylindre creux et que le premier applicateur contient deux électrodes (32a, 32b) capacitives opposées disposées dans le boîtier (26) sur des faces opposées de l'intérieur du boîtier (figure 2).

4. Appareil d'électrothérapie selon une des revendications 1 à 3, caractérisé en ce que l'oscillateur haute fréquence (16) contient un dispositif de modulation de l'amplitude d'une oscillation haute fréquence (28) produite par ses soins par une oscillation modulatrice ainsi qu'un dispositif de réglage d'une relation de phases prédonnée entre l'oscillation modulatrice et la deuxième oscillation basse fréquence (30).

5. Appareil d'électrothérapie selon la revendication 4, caractérisé en ce que l'oscillation modulatrice comporte des premiers domaines (28a) d'amplitude relativement faible destinés à produire une élévation de la température de base des tissus et de seconds domaines (28b) d'amplitude relativement élevée, alternant avec ces premiers domaines (28a), destinés à produire de brèves phases de suréchauffement.

6. Appareil d'électrothérapie selon la revendication 5, caractérisé en ce que les seconds domaines (28b) coïncident essentiellement avec les passages par zéro de la seconde oscillation basse fréquence (30).

7. Appareil d'électrothérapie selon l'une des revendications 1 à 6, caractérisé par un dispositif de synchronisation de l'oscillateur (14) destiné au signal basse fréquence avec l'onde du pouls d'un malade traité.

8. Appareil d'électrothérapie selon l'une des revendications 1 à 7, caractérisé par un dispositif de modulation de l'oscillateur haute fréquence (16) avec l'onde du pouls d'un malade traité.

FIG.1

NF-APPLIKATOR
18

HF·APPLIKATOR 20

NF—     HF—
GENERATOR
INDUKTIV

10          14        16

FIG.2

32a

12'

32b

ELEKTRISCHES
HF-FELD

NF-MAGNETFELD

NF—     HF—
GENERATOR
INDUKTIV | KAPAZITIV

14          16

30

NF-AMPLITUDEN
EXTREM NIEDERFREQUENT
VON 0,02 Hz — 20 Hz

HF-AMPLITUDEN ZUR
ERZEUGUNG EINER
ERHÖHTEN GEWEBE-
BASISTEMPERATUR BIS ≦ 40°C.

28      28a      28a      28b

FIG.3  HF-AMPLITUDEN ZUR
ERZEUGUNG VON KURZEN ÜBERWÄRMUNGSPHASEN ( > 40°C )